# EUROPEAN PATENT APPLICATION

(11) **EP 1 424 079 A1**
(43) Date of publication of application: **02.06.2004**
(21) Application number: 02026546.8
(22) Date of filing: 27.11.2002
(51) Int. Cl.: A61K 45/06, A61K 31/00, A61K 31/192, A61K 31/215, A61P 13/00

(54) **Combination of a beta-3-receptor agonist and of a reuptake inhibitor of serotonin and/or norepinephrine**

(71) Applicant: Boehringer Ingelheim International GmbH, 55216 Ingelheim am Rhein (DE)
(72) Inventor: Ebinger, Ursula, 97230 Estenfeld (DE); Mehlburger, Ludwig, 55411 Bingen (DE); Michel, Martin C., 1071 ED Amsterdam (NL); Wienrich, Marion, 64331 Weiterstadt (DE)

(57) **Abstract**

This invention describes a new combination for the treatment of urinary incontinence and urge urinary incontinence comprising a dual reuptake inhibitor of serotonine and/or - preferably and - norpinephrine and a beta-3-receptor agonist for the treatment of urinary incontinence.

## Description

### Background of the Invention

### 1. Field of the Invention

This invention describes a new combination for the treatment of urinary incontinence and urge urinary incontinence comprising a dual reuptake inhibitor of serotonine and/or - preferably and -norpinephrine and beta-3-receptor agonist, like Duloxetine and its pharmacologically acceptable salts and a beta-r-receptor agonist, like (-)-Ethyl 2-[4-(2-{[(1S,2R)-2-hydroxy-2-(4-hydroxyphenyl)-1-methylethyl]amino}-2,5 -dimethylphenyloxy]acetate and/or (-)-2-[4-(2-{[(1S,2R)-2-hydroxy-2-(4-hydroxyphenyl)-1-methylethyl]amino}-2,5-dimethylphenyloxy]acetate and/or pharmacologically acceptable salts thereof.

### 2. Technology Description

Since the early 1960s is known that tricyclic compounds being reuptake inhibitors of the catecholamines type have pharmacodynamic activities that impact the urinary function.
Such tricyclic compounds being reuptake inhibitors of all the catecholamines release in the synaptic cleft, thus resulting in prolongation and enhancement of the dopamine (DA), noradrenaline (NA) and serotonine (5-hydroxytryptamine = 5-HT) action. Lack of selectivity also causes undesired side effects particularly on the acetylcholine, and histamine mediated neurotransmission.

Another interesting class of compounds are selective norepinephrine reuptake inhibitors. Lower-than-normal levels of norepinephrine are associated with a variety of symptoms including lack of energy, motivation, and interest in life. Thus, a normal level of norepinephrine is essential to maintaining drive and capacity for reward. These neurotransmitters travel from the terminal of a neuron across a small gap (i. e., the synaptic cleft) and bind to receptor molecules on the surface of a second neuron. This binding elicits intracellular changes that initiate or activate a response or change in the postsynaptic neuron.

Inactivation occurs primarily by transport (i. e., reuptake) of the neurotransmitter back into the presynaptic neuron. Abnormality in noradrenergic transmission among others in neurological disorders.

A balanced dual reuptake inhibitor of serotonine and norpinephrine is Duloxetine, (+)-N-methyl-3- (1- naphthalenyloxy)-3- (2-thienyl) propanamine which is disclosed in U. S. Patent No. US 4,956,388 or US 5,023,269
The compound may be used to treat urinary incontinence as disclosed for example by US 5,744,474.

The compound is represented by the following formula I:

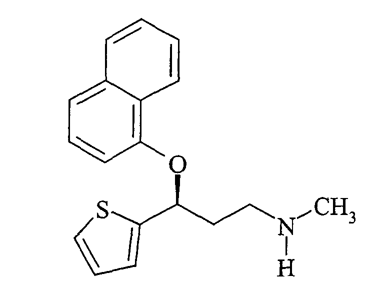

It may be administered as the hydrochloride salt and as the (+) enantiomer.

Beta-3-receptor agonists may be used to treat urinary incontinence, too as disclosed by EP 0958835. An interesting examples of a beta-3-receptor agonists are (-)-Ethyl 2-[4-(2-{[1S,2R)-2-hydroxy-2-(4-hydroxyphenyl)-1-methylethyl]amino}-2,5-dimethylphenyloxy]acetate or (-)-2-[4-(2-{[1S,2R9-2-hydroxy-2-(4-hydroxyphenyl)-1-methylethyl]amino}-2,5-dimethylphenyloxy] acetate, the other enantiomeres thereof and pharmacologically active salts thereof.

Both compounds are represented by the following formula II:

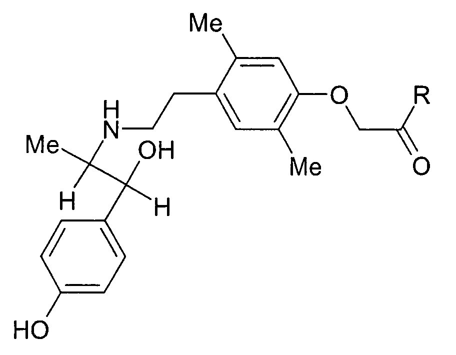

R = Ethoxy being (-)-Ethyl 2-[4-(2-{[1S,2R)-2-hydroxy-2-(4-hydroxyphenyl)-1-methylethyl]amino}-2,5-dimethylphenyloxy]acetate monohydrocloride
R= OH being (-)-2-[4-(2-{[1S,2R9-2-hydroxy-2-(4-hydroxyphenyl)-1-methylethyl]amino}-2,5-dimethylphenyloxy] acetate.

Despite the advances in the art as outlined in the corresponding aforementioned patent literature for each of the individual agonsits, it would be desirable to develop a pharmaceutical composition that would have both the benefits of the serotonine/norepinephrine reuptake inhibitors and that of the beta-3-receptor agonists.

### Brief Summary of the Invention

In accordance with the present invention a novel pharmaceutical composition is provided. More specifically, the composition combines one or more selective serotonine/norepinephrine reuptake inhibitors like Duloxetine with one or more beta-3-receptor agonists, preferably (-)-Ethyl 2-[4-(2-{[1S,2R)-2-hydroxy-2-(4-hydroxyphenyl)-1-methylethyl]amino-2,5-dimethylphenyloxy] acetate, (-)-Ethyl 2-[4-(2-{[1S,2R)-2-hydroxy-2-(4-hydroxyphenyl)-1-methylethyl] amino}-2,5-dimethylphenyloxy]acetate monohydrocloride, (-)-2-[4-(2-{[1S,2R9-2-hydroxy-2-(4-hydroxyphenyl)-1-methylethyl]amino} -2,5-dimethylphenyloxy]acetate or other pharmacologically acceptable salts thereof. The composition is considered to be particularly effective against incontinence and stress incontinence.

A first embodiment of the present invention provides a composition comprising: (a) a pharmaceutically effective amount of one or more serotonine and / or norepinephrine reuptake inhibitors or a pharmaceutically effective salt thereof; and (b) a pharmaceutically effective amount of one or more beta-3-receptor agonists or a pharmaceutically effective salt thereof.

In particularly preferred embodiments, component (a) comprises Duloxetine in either its enantiomeric or racemic form and component including the active metabolites thereof and (b) comprises (-)-Ethyl 2-[4-(2-{[1S,2R)-2-hydroxy-2-(4-hydroxyphenyl)-1-methylethyl]amino}-2,5-dimethylphenyloxy]acetate, (-)-Ethyl 2-[4-(2-{[1S,2R)-2-hydroxy-2-(4-hydroxyphenyl)-1-methylethyl]amino}-2,5-dimethylphenyloxy]acetate monohydrochloride, (-)-2-[4-(2-{[1S,2R9-2-hydroxy-2-(4-hydroxyphenyl)-1-methylethyl]amino}-2,5-dimethylphenyloxy]acetate or other pharmacologically acceptable salts thereof, including the active metabolites thereof.

Yet another embodiment of the present invention provides a method for treating or preventing urinary incontinence or diseases or disorders of the central nervous system being related with urinary incontinence comprising administering a therapeutically effective amount of the above composition to a mammal. In most instances, the mammal will be a human, and the disease or disorder to be treated is incontinence.

A further embodiment of the present invention comprises the use of the above composition to prepare a medicament for treating or preventing incontinence or diseases or disorders of the central nervous system being related to urinary incontinence.

An object of the present invention is to provide novel compositions having biological activity.

A further object of the present invention is to provide a method for treating or preventing incontinence or such related diseases of the central nervous system by using the novel compositions of the present invention.

An additional object of the present invention is to provide an effective treatment for incontinence.

Another objective is to provide a new effective treatment of such disorders which show synergistic efficacy versus the montherapies.

These, and other objects, will readily be apparent to those skilled in the art as reference is made to the detailed description of the preferred embodiment.

### Detailed Description of the Preferred Embodiment

In describing the preferred embodiment, certain terminology will be utilized for the sake of clarity. Such terminology is intended to encompass the recited embodiment, as well as all technical equivalents which operate in a similar manner for a similar purpose to achieve a similar result. To the extent that any pharmaceutically active compound is disclosed or claimed, it is expressly intended to include all active metabolites produced in vivo, and, is expressly intended to include all enantiomers, isomers or tautomers where the compound is capable of being present in its entantiomeric, isomeric or tautomeric form. Also included are pharmacologically acceptable salts thereof.

The present invention provides a novel composition which is a combination of different chemical entities, more specifically, the first entity being a serotonine and/or norepinephrine reuptake inhibitor and the second being a beta-3-receptor agonist.

The first component is a norepinephrine reuptake inhibitor, with selective norepinephrine reuptake inhibitors being particularly preferred. This list of compounds includes, but is not limited to the following : tandamine, pirandamine, ciclazindol, fluparoxan, lortalamine, talsupram, talopram, prindamine, nomifensine, viloxazine, tomoxetine, duloxetine, venlafaxine, milnacipran and reboxetine, with reboxetine being particularly preferred.

Examples of pharmaceutically effective salts for the selective norepinephrine reuptake inhibitor include, but are not limited to salts prepared from pharmaceutically acceptable acids or bases, including organic and inorganic acids and bases. When the preferred compound of use is basic (for example reboxetine), salts may be prepared from pharmaceutically acceptable acids. Suitable pharmaceutically acceptable acids include acetic, benzenesulfonic (besylate), benzoic, p-bromophenylsulfonic, camphorsulfonic, carbonic, citric, ethanesulfonic, fumaric, gluconic, glutamic, hydrobromic, hydrochloric, hydroiodic, isethionic, lactic, maleic, malic, mandelic, methanesulfonic (mesylate), mucic, nitric, oxalic, pamoic, pantothenic, phosphoric, succinic, sulfuric, tartaric, p-toluenesulfonic, and the like. Examples of such pharmaceutically acceptable salts include, but are not limited to, acetate, benzoate, hydroxybutyrate, bisulfate, bisulfite, bromide, butyne-1, 4-dioate, carpoate, chloride, chlorobenzoate, citrate, dihydrogenphosphate, dinitrobenzoate, fumarate, glycollate, heptanoate, hexyne-1, 6-dioate, hydroxybenzoate, iodide, lactate, maleate, malonate, mandelate, metaphosphate, methanesulfonate, methoxybenzoate, methylbenzoate, monohydrogenphosphate, naphthalene-1-sulfonate, naphthalene-2-sulfonate, oxalate, phenylbutyrate, phenylproionate, phosphate, phthalate, phylacetate, propanesulfonate, propiolate, propionate, pyrophosphate, pyrosulfate, sebacate, suberate, succinate, sulfate, sulfite, sulfonate, tartrate, xylenesulfonate, and the like.

In particularly preferred embodiments the selective norepinephrine reuptake inhibitor is duloxetine, N-methyl-3- (1- naphthalenyloxy)-3- (2-thienyl) propanamine, and its pharmaceutically acceptable salts, in either its enantiomeric (particularly the (+) enantiomer) or racemic form and in most preferred cases the (+)-form.

The selection of the dosage of the first component is that which can provide relief to the patient.

As is well known, the dosage of this component depends on several factors such as the potency of the selected specific compound, the mode of administration, the age and weight of the patient, the severity of the condition to be treated, and the like. This is considered to be within the skill of the artisan and one can review the existing literature on the components to determine optimal dosing. To the extent necessary for completion, the synthesis of the components and dosages described in the patents or CAS documents referenced in the Technology Description portion of this document are expressly incorporated by reference
Desirably, when Duloxetine is selected as the active agent, the daily dose contains from about 0. 1 mg. to about 500 mg. More preferably, each dose of the component contains about 0.5 to about 8 mg of the active ingredient. This dosage form permits the full daily dosage to be administered in one or two oral doses. This will allow for final formulations containing 0.1, 0.2, 0.3, 0.4, 0.5, 0.6, 0.7, 0.8, 0.9, 1.0, 1.1, 1.2, 1.3, 1.4, 1.5, 1.6, 1.7, 1.8, 1.9.2.0, 2.1, 2.2, 2.3, 2.4, 2.5, 2.6, 2, 7, 2.8, 2.9, 3.0, 3.1, 3.2, 3.3, 3.4, 3.5, 3.6, 3.7, 3.8, 3.9.4.0, 4.1, 4.2, 4.3, 4.4, 4.5, 4.6, 4, 7, 4.8, 4.9, 5.0, 5.1, 5.2, 5.3, 5.4, 5.5, 5.6, 5, 7, 5.8, 5.9, 6.0, 6.1, 6.2, 6.3, 6.4, 6.5, 6.6, 6.7, 6.8, or 6.9 mg of active.

More than once daily or twice daily administrations (e. g., 3,4,5 or 6 administrations per day) are also expressly contemplated herein.

The average daily adult dosage of the other norepinephrine reuptake inhibitors is as follows. The dosages expressly include all numerical values, whole or fractional, within the stated range. Pediatric dosages may be less.

Component Average Daily Dosage (mg/day/patient)
Tandamine 7. 5 to 3750
Pirandamine 7. 5 to 3750
Ciclazindol 5 to 500
Fluparoxan. 75 to 750
Lortalamine 1 to 200
Talsupram 1 to 3750
Talopram 1 to 3750
Prindamine 1 to 3750
Nomifensine 1 to 80
Viloxazine 1 to 3750
Tomoxetine 1 to 200
Duloxetine 5 to 500
Venlafaxine 2 to 200
Milnacipran 7. 5 to 75

The second component comprises one or more beta-3-receptor agonist. Preferred are beta-3-receptor agonist of the catecholamine-type. Most preferred are (-)-Ethyl 2-[4-(2-{[1S,2R)-2-hydroxy-2-(4-hydroxyphenyl)-1-methylethyl]amino}-2,5-dimethylphenyloxy]acetate or (-)-2-[4-(2-{[1S,2R9-2-hydroxy-2-(4-hydroxyphenyl)-1-methylethyl]amino}-2,5-dimethylphenyloxy] acetate, the other enantiomeres thereof and pharmacologically active salts thereof. The compounds are disclosed by WO 00/02846.

As is well known, the dosage and administrative regimen (i. e., one, two, three or more administrations per day) of the second component depends on the factors referred to in connection with the dosage selection of the first component. To the extent necessary for completion, the synthesis of the components and dosages described in the patents or CAS documents referenced in the Technology Description portion of this document are expressly incorporated by reference. The average adult daily dosage of the second component is from about 10 mg to about 750mg per day, administered in one or more doses. The dosages expressly include all numerical values, whole or fractional, within the stated range. Pediatric dosages may be less.

Compositions of the present invention can conveniently be administered in a pharmaceutical composition containing the active components in combination with a suitable excipient. Such pharmaceutical compositions can be prepared by methods and contain excipients which are well known in the art. A generally recognized compendium of such methods and ingredients is Remington's Pharmaceutical Sciences by E. W.

Martin (Mark Publ. Co., 15th Ed., 1975). To the extent necessary for completion, this reference is hereby incorporated by reference. The compositions of the present invention can be administered parenterally (for example, by intravenous, intraperitoneal, subcutaneous or intramuscular injection), topically, orally, intranasally, intravaginally, or rectally, with oral administration being particularly preferred.

For oral therapeutic administration, the inventive composition may be combined with one or more excipients and used in the form of ingestible tablets, buccal tablets, troches, capsules, elixirs, suspensions, syrups, wafers, chewing gums, foods and the like. Such compositions and preparations should contain at least 0.1 % of active compound. The percentage of the compositions and preparations may, of course, be varied and may conveniently be between about 0.1 to about 100% of the weight of a given unit dosage form. The amount of active compound in such therapeutically useful compositions is such that an effective dosage level will be obtained.

The tablets, troches, pills, capsules, and the like may also contain the following : binders such as gum tragacanth, acacia, corn starch or gelatin; excipients such as dicalcium phosphate; a disintegrating agent such as corn starch, potato starch, alginic acid and the like ; a lubricant such as magnesium stearate; and a sweetening agent such as sucrose, fructose, lactose or aspartame or a flavoring agent such as peppermint, oil of wintergreen, or cherry flavoring. The above listing is merely representative and one skilled in the art could envision other binders, excipients, sweetening agents and the like. When the unit dosage form is a capsule, it may contain, in addition to materials of the above type, a liquid carrier, such as a vegetable oil or a polyethylene glycol. Various other materials may be present as coatings or to otherwise modify the physical form of the solid unit dosage form. For instance, tablets, pills, or capsules may be coated with gelatin, wax, shellac or sugar and the like. A syrup or elixir may contain the active compound, sucrose or fructose as a sweetening agent, methyl and propylparabens as preservatives, a dye and flavoring such as cherry or orange flavor.

Of course, any material used in preparing any unit dosage form should be pharmaceutically acceptable and substantially non-toxic in the amounts employed. In addition, the active components may be incorporated into sustained-release preparations and devices including, but not limited to, those relying on osmotic pressures to obtain a desired release profile. Once daily formulations for each of the active components are specifically included.

The inventive composition, containing the two active components, may be administered in the same physical form or concomitantly according to the above described dosages and in the above-described delivery vehicles. The dosages for each active component can be measured separately and can be given as a single combined dose or given separately. They may be given at the same or at different times as long as both actives are in the patient at one time over a 24-hour period. Concomitant or concurrent administration means the patient takes one drug within about 5 minutes of taking the other drug. Because the goal is to provide rapid symptomatic relief to the patient, in most cases when treatment is started the two drugs would be administered to the patient close in time and typically concomitantly; thereafter, the timing of each drug's administration may not be as important.

The inventive composition is used to treat incontinence or any of the diseases or disorders of the central nervous system. Such diseases and disorders are defined in
The Diagnostic and Statistical Manual of Mental Disorders-IV (DSM-IV) (American
Psychiatric Association (1995)). To the extent necessary for completion, the contents of this reference and all of the defined diseases or disorders are expressly incorporated by reference. Also considered is the treatment of incontinence of any type.

Representative diseases or disorders include, but are not limited to the following : incontinence, a stress induced problem with the urinary incontinence, stress incontinence, genuine stress incontinence, and mixed incontinence and other forms of urinary incontinence. Stress urinary incontinence is a symptom describing involuntary loss of urine on carrying out any activity that raises intra-abdominal pressure such as coughing or sneezing. Stress incontinence is also a clinical sign, that is the observation by a care giver of a jet of urine escaping from the urethral meatus (opening) when the patient coughs or strains.

Genuine Stress Incontinence (urge incontinence) is the pathological diagnosis of an incompetent urethral sphincter as diagnosed by Urodynamic testing. Mixed incontinence is stress incontinence in combination with urge incontinence. The latter is a part of the symptom complex of the Overative Bladder. Retention may be due to outflow obstruction (e. g., high urethral pressure), poor detrusor (bladder muscle) contractility or lack of coordination between detrusor contraction and urethral relaxation. The inventive drug combination can be used in connection with stress incontinence, urge incontinence or mixed incontinence.

Treatment of the above diseases or disorders is accomplished by delivering a therapeutically effective amount of the inventive composition to a mammal. In most cases this will be a human being, but treatment of food animals (e. g., livestock and poultry) and companion animals (e. g., dogs, cats and horses) is expressly covered herein.

The novel composition is expected to provide rapid relief to those suffering from the above diseases or disorders with a minimal amount of deleterious side effects.

The invention is described in greater detail by the following non-limiting example.

In particular preferred combinations are
Duloxetine and (-)-Ethyl 2-[4-(2-{[(1S,2R)-2-hydroxy-2-(4-hydroxyphenyl)-1-methylethyl]amino}-2,5-dimethylphenyloxy]acetate.
Duloxetine and (-)-Ethyl 2-[4-(2-{[(1S,2R)-2-hydroxy-2-(4-hydroxyphenyl)-1-methylethyl]amino} -2,5-dimethylphenyloxy] acetate monohydrochloride.
Duloxetine (-)-2-[4-(2-{[(1S,2R)-2-hydroxy-2-(4-hydroxyphenyl)-1-methylethyl]amino}-2,5-dimethylphenyloxy] acetate
Duloxetine and (-)-2-[4-(2-{[(1S,2R)-2-hydroxy-2-(4-hydroxyphenyl)-1-methylethyl]amino}-2,5-dimethylphenyloxy]acettic acid monohydrochloride

Having described the invention in detail and by reference to the preferred embodiments thereof, it will be apparent that modifications and variations are possible without departing from the scope of the appended claims.

## Claims

1. A composition comprising: (a) a pharmaceutically effective amount of one or more serotonine and/or a norepinephrine reuptake inhibitors or a pharmaceutically effective salt thereof, preferraply serotonine and a norepinephrine reuptake inhibitors or a pharmaceutically effective salt thereof; and (b) a pharmaceutically effective amount of one or more beta-3-receptor agonist or a pharmaceutically effective salt thereof.

2. The composition according to claim 1 wherein component (a) is selected from the group consisting of tandamine, pirandamine, ciclazindol, fluparoxan, lortalamine, talsupram, talopram, prindamine, nomifensine, viloxazine, tomoxetine, duloxetine, venlafaxine; milnacipran and reboxetine and mixtures thereof and wherein component (b) is selected from the group consisting of tolterodine, propiverine, oxybutynin, trospium, darifenacin, temiverine and ipratropium and mixtures thereof.

3. The composition according to claim 2 wherein component (a) is Duloxetine in either its racemic or + (+) enantiomeric form and component (b) is (-)-Ethyl 2-[4-(2-{[(1S,2R)-2-hydroxy-2-(4-hydroxyphenyl)-1-methylethyl]amino)-2,5-dimethylphenyloxy]acetate and/or (-)-2-[4-(2-{[(1S,2R)-2-hydroxy-2-(4-hydroxyphenyl)-1-methylethyl]amino}-2,5-dimethylphenyloxy] acetate and/or pharmacologically acceptable salts thereof.

4. The composition according to claim 3 containing between about 0.1 mg to about 500 mg Duloxetine and between about 10 mg to about 750 mg of component (b).

5. The composition according to claim 1 wherein component (a) and component (b) are maintained in the same delivery vehicle.

6. The composition according to claim 1 wherein component (a) and component (b) are maintained in different delivery vehicles.

7. A method for treating urinary incontinence or a disease or disorder of the central nervous system being related to urinary incontinence in a mammal comprising administering to said mammal a pharmaceutically effective amount of a composition comprising: (a) a pharmaceutically effective amount of a component (a) as defined in claim 1 and (b) a pharmaceutically effective amount of a component (b) as defined in claim 1).

8. The method according to claim 7 wherein said disease or disorder is selected from the group consisting of urinary incontinence, stress incontinence, genuine stress incontinence, and mixed incontinence and other forms of urinary incontinence.

9. The method of claim 7 wherein said composition is administered rectally, topically, orally, sublingually, intranasally, transdermally or parenterally.

10. The method of claim 7 wherein component (a) and component (b) of said composition are simultaneously administered.

11. The method of claim 7 wherein component (a) and component (b) of said composition are concomitantly administered.

12. The method according to claim 7 wherein said disease or disorder comprises incontinence.

13. The method according to claim 12 wherein said incontinence is stress incontinence, genuine stress incontinence or mixed incontinence.

14. The method according to claim 13 wherein component (a) of said composition comprises Duloxetine in the racemic or enantiomeric form and component (b) as defined under component (b) of claim 3.

15. The method according to claim 12 wherein said composition contains between about 0.1 mg to about 500 mg Duloxetine and between about 10 mg to about 750 mg component (b) per day.

16. A composition consisting essentially of: (a) a pharmaceutically effective amount of Duloxetine racemic or enantiomeric form or a pharmaceutically effective salt thereof ; and (b) a component as defined under component (b) of claim 3, its active metabolites or combinations thereof or a pharmaceutically effective salt thereof; wherein components (a) and (b) are maintained in the same or in different delivery vehicles.

17. The use of a composition comprising: (a) a component as defined under component (a) of claim 1 or claim 2 and (b) a component as defined under component (b) of claim 1 or claim 2 to prepare a medicament for treating or preventing urinary incontinence.

18. A composition comprising: (a) a pharmaceutically effective amount of one or more components as defied under component (a) of claim 1; and (b) a pharmaceutically effective amount of one or more one or more components as defied under component (b) of claim 1 for use as a medicament.
